# EUROPEAN PATENT SPECIFICATION

(11) **EP 1 427 458 B1**
(45) Date of publication and mention of the grant of the patent: **24.11.2010**
(21) Application number: 02765028.2
(22) Date of filing: 17.09.2002
(51) Int. Cl.: A61L 33/00, A61L 33/06, A61L 31/10, A61L 29/08

(54) **TREATING SURFACES TO ENHANCE BIO-COMPATIBILITY**
OBERFLÄCHENBEHANDLUNG ZUR BIOKOMPATIBILITÄTSVERBESSERUNG
TRAITEMENT DE SURFACES POUR L'AMELIORATION DE LA BIOCOMPATIBILITE

(30) Priority: 17.09.2001 GB 0122393
(43) Date of publication of application: 16.06.2004
(73) Proprietor: Bayer MaterialScience AG, 51368 Leverkusen (DE)
(72) Inventor: AL-LAMEE, Kadem Gayed, Leeds, Yorkshire LS16 7AD (GB); LOTT, Martyn Peter, Sheffield, Yorkshire S36 9UY (GB); COOK, Diane, Huddersfield, West Yorkshire HD5 0LH (GB); BAYES, Stuart, Leeds, West Yorkshire LS27 8QZ (GB)
(86) International application number: PCT/GB2002/004227
(87) International publication number: WO 2003/024500

(56) References cited:
- EP-A- 0 608 095
- WO-A-98/55162
- US-A- 4 373 009
- US-A- 4 872 867
- US-A- 5 221 724
- US-A- 5 430 121
- US-A- 6 013 855
- US-A- 6 096 525

## Description

This invention relates to a method of treating a stent or other metal, glass or ceramics article having at its surface oxide or hydroxide to enhance the bio-compatibility and/or physical characteristics of the surface

EP-A-0433011 discloses that since the mid-to late 1980s, intra-arterial stents had found extensive use as a treatment to prevent restenosis subsequent to balloon angioplasty or atherectomy. A recurrent problem was (and continues to be) that excessive tissue growth (intimal hyperplasia) at the site of the balloon dilation or atherectomy plaque excision results in restenosis of the artery. One possible solution to this problem (US-A-4768507) had been to coat the stent with an anti-thrombogenic surface so as to reduce platelet fibrin deposition. But although an anti-thrombogenic coating can prevent acute thrombotic arterial closure and decrease the need for anticoagulant drug therapy, there is still an urgent need to decrease restenosis, which is caused by intimal hyperplasia.

It is well known that radiation therapy can reduce the proliferation of rapidly growing cancer cells in a malignant tumour, and in EP-A-0433011 use was made of this property by providing a stent comprising a tubular structure insertable into an artery and locatable therein to maintain the lumen of the artery patent, wherein the stent comprises or is constructed of a material that is radioactive. In EP-A-0566245 it was reported that an intraluminal stent comprising fibrin is capable of reducing the incidence of restenosis at the site of a vascular injury and can also serve as a matrix for the local administration of drugs to the site of a vascular injury. EP-A-0701802 disclosed a drug eluting intravascular stent comprising: (a) a generally cylindrical stent body; (b) a solid composite of a polymer and a therapeutic substance in an adherent layer on the stent body; and (c) fibrin in an adherent layer on the composite.

US-A-5356433 discloses the treatment of a stent or other medical device by the alleged formation of covalent linkages between a biologically active agent and a metallic surface. In one example tantalum stents were primed with a solution in ethanol of *N*-(2-aminoethyl-3-aminopropyl)trimethoxysilane so that a bond was formed between the tantalum oxide layer on the surface of the stents and the silicon of the silane on curing at 110°C. Heparin is then coupled to the amino groups using 1,3-ethyldimethyl-aminopropyl carbodiimide hydrochloride (EDC). In a second example, an ethanolic solution of an aminofunctional polymeric silane, trimethylsilylpropyl substituted polyethylenediamine is bonded to the surface of tantalum stents, also with curing at 110°C, after which heparin was coupled to the coating using EDC. Other examples use stainless steel wire, platinum tungsten wire and aminopropyl-trimethoxysilane as primer. However, priming has to be carried out with heating.

The present applicants have found experimentally, as described below, that covalent bonds to the metal surface are not formed under the conditions described. This is believed to be because the water, which is inevitably present in the ethanol, hydrolyses the linkages between the methoxy groups and silicon and because the reaction between the trimethoxysilane groups and surface oxide requires a catalyst that is absent.

US 6,013,855 (United States Surgical) discloses a method of attaching hydrophilic polymers to the surface of an article having a plurality of hydroxyl or oxide groups attached thereto. The method involves exposing the surfaces to a silanated hydrophilic polymer, for example (RO)₃SiR'(-urea link-)PVA, dissolved in a 95:5 alcohol to water solution. As an alternative to PVA, a natural polymer such as dextran can be used. As mentioned above in relation to US-A-5356433, it is believed that the use of an aqueous alcoholic solvent does not result in covalent bonds with the article surface. Also, the fact that the polymer and silane are coupled prior to reaction with the article surface means that it is difficult to control the amount of polymer attached to the surface. This is because the oxide and hydroxide groups on the surface are not particularly accessible, making it difficult to couple the silanated polymer thereto.

US 6,248,127 (Medtronic AVE, Inc.) discloses a biocompatible coating comprising a silane having isocyanate functionality to which a biocompatible molecule such as heparin can be attached. Optionally, a linking group such as an organic chain can be present between the silane and the isocyanate group. The coating can be applied in a single layer and a primer is not required.

US 6,387,450 (Medtronic AVE, Inc.) relates to a coating composition comprising hyaluronic acid or a salt thereof and a blocked polyisocyanate in a solvent comprising water.

US 5,053,048 (Cordis Corporation) discloses a thromboresistant coating comprising a copolymer of aminosilane or aminosiloxane and a silane which is not an amino silane. This mixture forms a three dimensional matrix on the surface of the base substrate and an antithrombogenic bioactive such as heparin is then attached to the substrate via the coating. The coating is dried at high humidity, and it is believed therefore that the water present causes hydrolysis of the alkoxy/silicon bonds. Also, the reaction is carried out in the absence of any catalyst for promoting the formation of covalent bonds between the surface oxide/hydroxide groups and the alkoxysilane.

The present applicants have previously disclosed in WO 98/55162 a method of treating stent or other metal, glass or ceramics article having at its surface oxide or hydroxide to enhance the bio-compatibility and/or physical characteristics of the surface, said method comprising the steps of priming said surface by means of functional molecules each of which has at least one alkoxysilane group which can form at least one first covalent bond by reaction with the oxide or hydroxide of said surface and at least one other group which can participate in free-radical polymerisation, the priming being carried out by contacting said surface in an aprotic organic solvent with said functional molecules and with an acid catalyst for forming said first covalent bond; and forming chains covalently attached to said other group of the functional molecules by free-radical polymerisation of at least one polymerizable monomer which imparts hydrophilic properties to said chains.

It is an object of the invention to provide a simpler process for forming an anti-thrombogenic and/or anti-restenosis layer on a stent or other oxide-coated implantable article that is simpler to use than in the prior art and which does not require free-radical polymerisation.

That problem is addressed, according to the invention by a method of treating an article having at its surface oxide or hydroxide, said method comprising the steps of priming said surface by contact with an alkoxysilane in an aprotic organic solvent in the presence of an acid catalyst so that the alkoxysilane molecules react with the oxide or hydroxide of said surface to form covalent bonds, the alkoxysilane comprising one or more amino, hydroxyl, carboxylic acid or acid anhydride groups; and covalently coupling a polymer to said primed surface via said alkoxysilane..

The article that is to be treated according to the invention may be of stainless steel or nitanol. It may be a coronary stent (endovascular prosthesis), peripheral stent, heat exchanger used in conjunction with biological material, guide wire used in angioplasty, artificial heart valve, device is used for storage and/or transfer of biological material or other medical device. The stent may be of any of the following types: a coil spring stent; a thermal shaped memory alloy stent; a self-expanding steel spiral stent; a self-expandable stainless steel mesh stent; or a balloon expanding stent comprising inter-digitating coils.

Prior to priming the surface of the article should be cleaned to remove grease and other contaminants. A suitable cleaning procedure involves treatment with aqueous alkali, e.g. NaOH with sonication, followed by rinsing with water and oven drying.

The priming step involves contacting the article with alkoxysilane in an aprotic organic solvent, for example toluene, in the presence of an acid catalyst which will normally be an organic acid that is compatible with and can dissolve in the aprotic organic solvent, catalyst, for example glacial acetic acid, followed by rinsing in fresh aprotic organic solvent to remove unreacted material, after which drying is carried out at an elevated temperature e.g. about 50-55°C and preferably under vacuum. Further washing is carried out after drying using the aprotic organic solvent followed by a water-miscible organic solvent and finally with deionised water. The intermediate solvent assists in the removal of hydrolysis by-products of the alkoxysilane. The use of low temperatures is important to stability, and the structure of nitanol, in particular, which is used for self-expanding stents, is vulnerable to changes in structure leading to degradation in properties if heated

significantly above 55°. The purpose of the priming step is to produce a monolayer rather than a coating of the functionalising agent on the oxide film of the metal.

Priming agents used may include alkoxysilanes of the formula (RO)₃Si(R¹X) wherein R represents methyl or ethyl and R¹ represents C₂-C₁₀ alkyl in which one or more methylene groups may be replaced by -NH- or -0-, C₂-C₁₀ cycloalkyl or cycloalkylalkyl, C₂-C₁₀ aralkyl or monocyclic or bicyclic aryl and X represents amino, hydroxyl, carboxylic acid or acid anhydride. Preferably R¹ represents C₂-C₁₀ alkyl in which one or more of the methylene groups is optionally replaced by -NH- and X represents -NH₂, and an example of a suitable priming agent is N-(3-(trimethoxysilyl)propyl)-ethylenediamine.

Reaction of the remaining reactive groups of the alkoxysilane with the polymeric material or "bridge" in the following step may be indirect via a linking intermediate or direct.

In indirect reaction, for example, a hydroxy- or amino-terminated alkoxysilane may be reacted with a linking intermediate in the form of an aliphatic or aromatic diisocyanate e.g. hexamethylene diisocyanate so that the first isocyanate group has formed a covalent bond with the hydroxy or amino functionality and the second isocyanate group is free and available to bond to hydroxy- or amino groups of the polymer bridge in a subsequent step. Such a reaction is easy to carry out by contact of the functionalized article with the diisocyanate in an aprotic organic solvent. It has the advantages that firstly the resulting adduct has highly reactive isocyanate groups which readily form covalent bonds with amino or hydroxyl groups of a 'bridging" polymer to be attached in a subsequent step, secondly that both the formation of the adduct and the reaction with the bridging polymer can be carried out under mild conditions and thirdly that the "spacer arms" which link silicon attached to oxide of the metal surface with the amino or other terminal functionality of the primer and which are provided e.g. by a chain of alkylene groups are further extended.

Where the bridging polymer is itself a biological active relatively large molecule, as in the case of heparin, for example, extension of the spacer arms improves the availability of the heparin or other large molecule and hence its biological effectiveness. Other linking intermediates with reactive terminal groups may be used, for example a di-epoxy compound which will react with a range of terminal groups of the oxide-bound alkoxysilane and with a wide range of groups in intended bridging polymers. A further possibility in indirect reaction is to activate the terminal group, e.g. by converting terminal amino to terminal isocyanate by reaction with thionyl chloride.

In the direct reaction alternative, the terminal group of the alkoxysilane may undergo condensation with available groups of the bridging polymer, for example an amide or ester-forming reaction. Thus an alkoxysilane that is hydroxy- or amino-terminated may be reacted with a bridging polymer having available carboxyl groups, e.g. carboxymethyl cellulose. Correspondingly an alkoxysilane that is terminated by carboxyl or by acid anhydride may be reacted with hydroxyl groups of the intended bridging polymer.

The function of the bridging polymer which is at least an oligomer is firstly to provide sites which can become covalently attached to the reactive groups of the alkoxysilane either directly or through an intermediate group as described above, and also to provide coupling sites for the biologically active material to be added later on. Each molecule of bridging polymer is relatively large compared to the alkoxysilane and has a multiplicity of coupling sites, so that the use of the bridging polymer enables a relatively high amount of the biologically active material to be attached with some stability to the stent, for example so that it becomes released only slowly into physiological fluids and has slow release properties when *in situ* in the body.

Carbohydrates comprise a class of polymers that are suitable for use in the invention and may include polysaccharide oligomers and polymers. Chemically modified celluloses e.g. carboxymethyl cellulose (CMC) is a suitable material and may be used e.g. in molecular weights of 5000 - 1,000,000, preferably 150,000-500,000. Because of the viscosity of aqueous solutions of carboxymethyl cellulose, relatively dilute solutions are used and, for example, a functionalised stent may be rotated in a solution of 0.05 wt% of CMC sodium salt. We have found that a strong bond is achieved, the carboxymethyl cellulose which is a highly water-soluble material remaining present on the stent or other functionalised oxide-coated material under prolonged washing e.g. for 72 hours at room temperature. CMC has the advantage that it becomes gradually hydrolysed in the body and therefore inherently has the property of releasing any biologically active material coupled to it. Other polysaccharides can also be used, for example dextran or naturally occurring polysaccharides.

One material that may be used is heparin, which is a naturally occurring substance that consists of a polysaccharide with a heterogeneous structure and a molecular weight ranging from approximately 6000 to 30000 Dalton (atomic mass units). It prevents uncontrolled clotting by suppressing the activity of the coagulation system through complexing with antithrombin (III), whose activity it powerfully enhances. Approximately one in three heparin molecules contains a sequence of highly specific structures to which antithrombin binds with high affinity. When bound to the specific sequence, the coagulation enzymes are inhibited at a rate that is several orders of magnitude higher than in the absence of Heparin. Thus, the heparin molecule is not in itself an inhibitor but acts as a catalyst for natural control mechanisms without being consumed during the anticoagulation process. The catalytic nature of heparin is a desirable property for the creation of a bioactive surface, because the immobilised heparin is not functionally exhausted during exposure to blood but remains a stable active catalyst on the surface. In addition to acting as a polysaccharide and an anti-clotting agent, heparin also offers sites for the attachment of small biologically active molecules.

Other non-carbohydrate polymers having available reactive groups such as -OH and - COOH can also be used, for example polyacrylic acid sodium salt having a molecular weight of 2000 or above and polyvinylalcohol. Hyperbranched polymers may also be used, see Anders Hult et al., Adv. Polymer Sc.., 143 (1999) pp. 1-34, the end groups being selected to be reactable with the alkoxysilane adhered to the oxide layer of the substrate.

Various biologically active materials may be attached to the bridging polymer. Such materials may include a second polymer that it covalently bonded to or ionically attracted to the bridging polymer via active sites. The second polymer may itself carry a biologically active compound that may be the same as or different from a small molecule active compound attached to the bridging polymer covalently or by ionic attraction. For example, if the bridging polymer does not itself have anti-thrombogenic properties, then there may be bonded thereto an anti-thrombogenic agent that may be an anticoagulant or an antiplatelet agent.

Suitable anti-coagulants include heparin, and hirudin, and there may also be used as antiplatelet agent a prostaglandin or analog thereof. Thus heparin may be attached to a stent or other implantable device that firstly has been functionalized with alkoxysilane and secondly has attached thereto a bridging polymer that is carboxymethyl cellulose or other carbohydrate. The heparin may be in modified form e.g. as described in our WO 98/55162 and may be attached to a carbohydrate or other bridging polymer using, for example, a di-epoxy or di-isocyanate linker which is reacted first with sites on the bridging polymer and second with sites on the heparin or derivative.

Also attachable to the bridging polymer is a compound that inhibits smooth cell proliferation and restenosis, for example mitoxantrone or a pharmaceutically acceptable salt thereof, paclitaxel (Taxol) or an analog thereof such as docetaxel (Taxotere), Taxane being used in the Quanam drug eluting stent which has been the subject of clinical trials, see also C. Herdeg et al., Semin. Intervent. Cardiol. 1998: 3, pp. 197-199, rapamycin or actinomycin D. Coupling of both an anti-coagulant such as heparin or hirudin and an inhibitor of smooth cell proliferation is expected to give very good response in both the short and longer term.

Use of radiolabelled materials as anti-proliferative agents is also possible. Attachment may be achieved by simply contacting the substrate with a solution of the biologically active material or materials, and allowing affinity between the biologically active compound and the polymer to bring about the required deposition of the active compound on the substrate. An advantage of this arrangement is that the biologically active compound is then available for local delivery and gradual release at the site where it is required.

The invention is further illustrated in the following examples.

### Example 1

### 1 Cleaning.

A commercially available stainless steel stent on a holder was placed into a vessel containing 0.1M aqueous NaOH. It was placed in an ultrasonic bath (Ultrawave U50 supplied by Ultrawave Limited of Cardiff UK) and sonicated for 15 minutes, rinsed briefly in deionised water followed by further sonication for 15 minutes in fresh deionised water. After a final brief rinse with deionised water the sample was dried for 60 hours at 130°C in an oven and allowed to cool in a dry atmosphere.

### 2 Functionalisation

The cooled sample was placed on a spindle holder attached to an overhead stirrer, and immersed in a solution of 10 drops of glacial acetic acid in 190 g of toluene in a measuring cylinder. A nitrogen line and a Parafilm (a thin transparent self-clinging film) cover were fitted to the cylinder to provide a nitrogen blanket above the toluene solution. With the stirrer rotating the spindle at a low speed, 9.5 ml of N(3-(trimethoxysilyl)propyl)-ethylenediamine (TMSPEA) (Sigma Aldrich Chemical Co) was injected by syringe through the nitrogen blanket into the toluene solution, after which the stirring continued for 15 minutes. The nitrogen line and Parafilm cover were then removed, after which the toluene reaction solution was replaced with toluene, the sample was rotated in this mixture for 15 minutes to remove any excess reagents, and dried at 50°C under vacuum (0.9 bar) for 24 hours. It was then rinsed further with a series of solvents: toluene, methanol, and deionised water, rotating the samples on a holder in the solvent for about 15 minutes each using an overhead stirrer.

### 3 Carboxymethylcellulose Coupling

Reaction Solution A was prepared and comprised 150 g of a 0.5% by weight solution of Blanose 7H3 SXF (carboxymethylcellulose, Honeywill & Stein Ltd, Sutton, Surrey, UK) in deionised water, to which was added 0.045 g of 1-ethyl-3-(3-dimethylaminopropyl) carbodiimide hydrochloride (Sigma Aldrich Chemical Co) with stirring. This solution was then acidified with 1M HCl to a pH between 5 to 6. After acidification the solution was left stirring for 30 minutes, with pH monitoring, after which it was ready for use.

The sample from functionalisation, still on a spindle, was fitted to an overhead stirrer and immersed in reaction solution A, after which and the sample holder was rotated for about 4 hours. The sample was then rinsed in de-ionized water for a period of one hour with rotating by means of the stirrer, and with change of the rinse water every 15 minutes, after which the sample was allowed to drain.

### 4 Mitoxantrone coupling and Release.

A 0.01% solution of mitoxantrone (Sigma Aldrich Chemical Co) was made up in deionised water. The samples are each immersed in 4mls of the solution and left rolling on a Spiramix (Denley Spiramix 5) for ∼17 hours (Samples placed in 100*16mm R.B. tube clarified polypropylene supplied by Jencons PLC.). After this time they were rinsed in deionised water until there was no evidence of the mitoxantrone being removed in the water. 4 mls of phosphate buffered saline solution (PBS) was then pipetted into a clean sample tube and the sample added. The samples were left in this solution for 1 hour on the Spiramix, after which absorbances were recorded by spectrophotometer at 660 nm. The solutions were then transferred back into the appropriate sample tube and 5 drops of 1M hydrochloric acid added from a dropping pipette. The samples were left for 10 minutes rolling on the Spiramix, after which an absorbance reading was recorded. Further readings were obtained after 1 hour or more to give a value for complete release of mitoxantrone. The absorbances recorded for the release solutions at 660 nm gave an indication of the amount of mitoxantrone attached to the carboxymethylcellulose coating on each sample. By use of a calibration curve plotting known concentrations of mitoxantrone solutions against the absorbance of the solution at 660 nm, the mitoxantrone concentration of the release solution was determined and from this the amount of mitoxantrone attached to each device. An absorbance of 0.09 at 660 nm was obtained for the 1 hour release in phosphate buffered saline solution, and an absorbance of 0.17 for the complete release of Mitoxantrone. This equates to approximately 31 micrograms of mitoxantrone attached to the stent. The above results show that the majority of the mitoxantrone has become tightly bound to the stent so that it is likely to become released only slowly under physiological conditions, and also that the compound can be applied in amounts that are effective to retard or inhibit cell growth leading to restenosis.

### Example 2

A commercially available stainless steel stent was prepared as in Example 1 up to and including stage 2, and then coupled with poly(acrylic acid) partial sodium salt as described below:

### Poly(acrylic acid) Coupling

Reaction Solution B was prepared by making up 150g of a 0.5% by weight aqueous solution ofpoly(acrylic acid) partial sodium salt (Average Mw ∼2000 by GPC, sodium content 0.6% supplied as a 60% solution in water by Sigma Aldrich Chemical Co). The pH of the solution was adjusted to between 5 to 6 by addition of 0.1M aqueous NaOH. Then 0.21 g of 1-ethyl-3-(3-dimethylaminopropyl) carbodiimide hydrochloride (Sigma Aldrich Chemical Co) was added to the solution, and the solution was allowed to stand for 30 minutes, after which it was ready for use.

The sample from functionalization with TMSPEA, still on a spindle, was fitted to an overhead stirrer and immersed in reaction solution B and the sample holder rotated for about 4 hours. The sample was then rinsed in de-ionized water with rotation for 1 hour, changing the rinse water every 15 minutes. The rinsed sample was allowed to drain.

The sample was then processed as in section 4 of Example 1 to give an absorbance value of 0.037 at 660 nm when released for 10 minutes in 4 ml of phosphate buffered saline solution with 5 drops of 1 M HCl, which absorbance value equates to 7 micrograms of mitoxantrone attached to the stent. The above results demonstrate that polyacrylic acid can be used as an alternative to carboxymethylcellulose and that useful quantities of mitoxantrone or other useful materials can be coupled to the polyacrylic acid.

### Example 3

A stainless steel heat exchange tube was prepared as in Example 1 up to (and including) stage 2, and then coupled with heparin as detailed below.

### Heparin Coupling

Reaction Solution C was prepared by dissolving 0.9 g of heparin (Heparin Sodium, USP/EP/JP lyophilized, Celsus Laboratories Inc, Cincinnati, USA) in 149.1 g of deionised water. To this solution 0.045 g of 1-ethyl-3-(3-dimethylaminopropyl) carbodiimide hydrochloride (Sigma Aldrich Chemical Co) was added, after which the solution was stirred to dissolve the added material and its pH was adjusted with 1M HCl to between 5 and 6. The solution was allowed to stand, with pH monitoring, for 30 minutes, after which it was ready for use.

Samples from functionalisation with TMSPEA, still on a spindle, were fitted to an overhead stirrer and immersed in reaction solution C and the sample holder rotated for about 4 hours. The samples were then rinsed in de-ionized water, using the stirrer to rotate them, for 1 hour, changing the rinse water every 15 minutes. After rinsing the samples were allowed to drain and processed as in section 4 of Example 1 to give the release values and below. The complete release values equate to 31 and 36 micrograms of mitoxantrone attached to the heparin coated devices

| | 1 Hour PBS | PBS +HCl 10mins | PBS+HCl 2hours |
|---|---|---|---|
| Sample 1 | 0.035 | 0.172 | 0.164 |
| Sample 2 | 0.040 | 0.203 | 0.194 |

The above example demonstrates the coupling of heparin to functionalised devices.

### Example 4

Stainless steel heat exchange tubes which mimic stents were prepared in an identical manner to Example 1 up until the Carboxymethylcellulose coupling stage, after which three concentrations ofBlanose 7H3 SXF were prepared ( 0.1%, 0.05% and 0.025% by weight solutions of Blanose 7H3 SXF were prepared each in 150 mls) to which 0.03% 1-ethyl-3-(3-dimethylaminopropyl)carbodiimide hydrochloride was added and each acidified as in Example 1. The rest of the procedure was as Example 1. The absorbances of the release solutions were determined at 660 nm and the corresponding amount of mitoxantrone attached determined from a calibration graph. The values are tabulated below.

| 7H3 SXF concentration | 1 Hour PBS | 10 mins PBS+ dil HCl | > 1 hour PBS + dil HCl | Mitoxantrone attached (µg) |
|---|---|---|---|---|
| 0.1% | 0.23 | 0.57 | 0.60 | 110 |
| 0.05% | 0.21 | 0.52 | 0.54 | 98 |
| 0.025% | 0.14 | 0.37 | 0.39 | 71 |

The above results show that CMC can be used in relatively low concentrations which are less viscous and therefore have better physical characteristics for uniform penetration into the mesh or other interstices of a stent, without there being a commensurate reduction in the amount of active compound that can be coupled to the stent.

### Example 5

Example 1 was repeated with stainless steel heat exchange tubes retaining samples after each process (cleaning, functionalisation, and carboxymethylcellulose coupling). These samples were all stained with mitoxantrone as in section 4 of Example 1 and then the mitoxantrone was released in PBS for 1 hour and with added dilute hydrochloric acid for 10 minutes, taking absorbance readings on a UV/Vis spectrometer (see table below). The final release values were then converted to amount of mitoxantrone per device using a calibration chart. The results in the table below show that a significant increase in the drug uptake is seen for the carboxymethylcellulose treated devices:

| Sample | PBS 1 hour | PBS+ dilute HCl 10 mins | Mitoxantrone on tube (µg) |
|---|---|---|---|
| Cleaned | 0.02 | 0.03 | 5.5 |
| TMSPEA functionalised | 0.01 | 0.01 | 1.8 |
| Fully treated | 0.18 | 1.82 | 331 |

The above results show that minimal amounts of active material become attached unless both the functionalization and the CMC coupling procedures are followed.

### Example 6

Samples (stainless steel heat exchange tubes) were prepared as in Example 3 (except 30 minutes reaction time was used in functionalisation rather than the 15 minutes used in Example 1) up to the heparin coupling stage. The heparin coupling was performed at four different levels of 1-ethyl-3-(3-dimethylaminopropyl) carbodiimide hydrochloride (EDC) as detailed in the below table.

Reaction Solution C Compositions for Example 6.

| Reaction Solution | % w heparin | %w EDC |
|---|---|---|
| 1 | 0.6 | 0.03 |
| 2 | 0.6 | 0.09 |
| 3 | 0.6 | 0.15 |
| 4 | 0.6 | 0.21 |

Each reaction was carried out using the general method from Example 3, then taken through to mitoxantrone take up and release. The absorbances of the release solutions were used to determine the amount of mitoxantrone taken up by each device, as displayed in the table below:

| Reaction Solution | Mitoxantrone take up by device (µg) |
|---|---|
| 1 | 38 |
| 2 | 36 |
| 3 | 38 |
| 4 | 42 |

The above results show that the amount of mitoxantrone taken up by the device to which it is to be coupled is relatively insensitive to EDC/heparin ratio within the ranges tested.

A TMSPEA functionalised tube was retained after stage 2 of the process in this example so that the effectiveness of the reaction could be checked. This used a solution of Eosin Y sodium salt to couple with the amine group of the TMSPEA on the sample's surface to visibly show coverage and then the release of the Eosin Y and its spectrometric determination to determine the amount coupled.

### Eosin Y Coupling.

The sample was placed in a sample tube (100*16mum R.B. tubes clarified polypropylene, Jencons PLC) and rolled on the Spiramix (Denley Spiramix 5) in approximately 4-6 mls of a 0.4% aqueous solution of eosin Y sodium salt (Sigma Aldrich Chemical Co) for approximately 1 hour, after which the sample was rinsed several times with deionised water until no visible stain was seen in the rinse. Visually, the tube had a relatively uniform and moderately pink stain.

Once rinsing had been completed, the sample was placed in a 50 ml sample tube and 4mls of 0.1 M NaOH was pipetted into it, the sample tube was placed on the Spiramix and it was rolled for ∼ 5 minutes. 20 mls of deionised water was then pipetted into the solution and the absorbance of the resulting solution was recorded at 517 nm using a spectrophotometer. The absorbance value was then converted into an amount of eosin Y attached to the sample by using a calibration graph of absorbance readings for known amounts of eosin Y sodium salt. The absorbance reading for the release solution was 0.83 at 517 nm corresponding to 205 µg of eosin Y.

The above results show that the functionalization step had worked as intended and that a uniform coverage of the device (stent or tube) with eosin or other material to be coupled thereto could be achieved.

### Example 7

Example 3 was repeated using a commercially available stent and a heparin/EDC solution in the coupling stage of the composition used in Reaction Solution 1 of Example 6. The released stent showed a mitoxantrone attachment of 9 micrograms. The practical equivalence of a tube and a stent was confirmed.

### Example 8

Six samples (stainless steel heat exchanger tubes available from Polystan) were cleaned as in section 1 of Example 1. The samples were then immersed in a solution of 2 mls of TMSPEA in 98 g of (95%v/v) ethanol, which was stirred by means of a magnetic follower for 3 minutes. The samples were then removed, and placed in an oven at 110 °C for 10 minutes. The samples were removed from the oven and three were reserved while the other three were rinsed first in (95%v/v) ethanol for 15 minutes, followed by deionised water for 15 minutes, using a suitable holder fitted to an overhead stirrer to rotate the samples in each solvent. The samples were then treated using eosin Y sodium salt, which causes the staining of any amine functional groups present on the surface as described below.

### Eosin Y Coupling.

All six samples were placed in sample tubes (100*16mem R.B. tubes clarified polypropylene, Jencons PLC) and rolled on the Spiramix (Denley Spiramix 5) in approximately 4-6 mls of a 0.4% aqueous solution of eosin Y sodium salt (Sigma Aldrich chemical co) for approximately 1 hour. After this time the samples were rinsed several times with deionised water until no visible stain was seen in the rinses.

Once rinsing had been completed, two samples from the ethanol rinsed and unrinsed sets were placed in 50ml sample tubes and 4mls of 0.1 M NaOH was pipetted into each. The sample tubes were placed on the Spiramix and rolled for - 5 minutes. 20 mls of deionised water was then pipetted into each sample and the absorbance of the resulting solution was recorded at 517 nm using a Jenway 6305 UV/Vis spectrophotometer. The values recorded were then converted into amounts of eosin Y attached to the samples by using a calibration graph of absorbance readings for known amounts of eosin Y sodium salt. Visual examination of the remaining samples showed patchy staining with the ethanol rinsed sample having a few patches of weak staining and the unrinsed sample having patches of stain on the metal.

| Sample | Absorbance at 517 nm | Eosin Y attached (ug) |
|---|---|---|
| No Rinse 1 | 0.84 | 207 |
| No Rinse 2 | 0.97 | 239 |
| Rinsed 1 | 0.05 | 12 |
| Rinsed 2 | 0.04 | 10 |

The values for un-rinsed tubes were similar to those seen in Example 6, but were visually patchy. The above results, which were intended to illustrate the priming procedure of Example 1 of US 5356433, show that useful attachment is not obtained under these conditions and that the majority of the apparently bonded material is loosely attached and removed by simple rinsing.

### Example 9

Example 3 was repeated using a higher molecular weight polyacrylic acid salt (polyacrylic acid, sodium salt, average Mw ca. 30,000, Sigma Aldrich Chemical Co) in place of the previous one, and using heat exchange tubes as the sample devices.

Following complete release of mitoxantrone, as in Example 3, an absorbance reading of 0.33 at 660 nm was obtained for the release solution, corresponding to 60 micrograms of the drug. This showed that a range of molecular weights ofpoly(acrylic acid) could be used in the process to obtain useable levels of drug coupling.

### Example 10

A commercially available stainless steel stent was cleaned and functionalised following the method in sections 1 and 2 of Example 1.

A polymer (DK01) was then prepared as set out below.

### Procedure for synthesis of DK01

| Chemical | Supplier | Catalogue number |
|---|---|---|
| anhydrous toluene | Aldrich | 24,451 |
| poly(propylene glycol) tolylene diisocyanate terminated (PPGTDI) MW = 2,300 | Aldrich | 43,349-7 |
| poly(dimethylsiloxane) bis (3-amino propyl) terminated (PDMSBAP) MW = 27,000 | Aldrich | 48,169 |
| (3-aminopropyl)-trimethoxysilane | Aldrich | 28,177-8 |
| nitrogen | Air Products | |

| | | |
|---|---|---|
| 1. A solution of PDMSBAP (5.00 g) in anhydrous toluene (63.5 g) was made up. 2. A solution of PPGTDI (1.00 g) in anhydrous toluene (63.5 g) was made up. 3. The solution of PPGTDI was slowly added to the solution of PDMSBAP with mixing under a blanket of nitrogen. 4. The reaction mixture was allowed to mix for 90 mins and then (3-aminopropyl)-trimethoxysilane (0.75 g) was added. 5. The reaction solution was mixed for a further hour. | | |

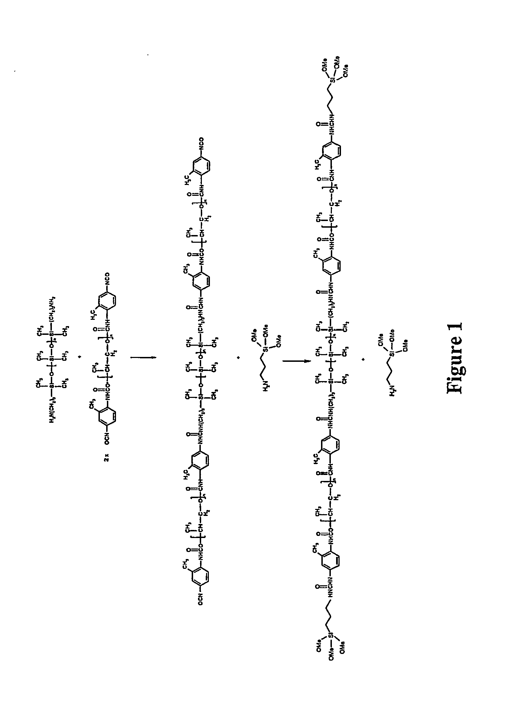

The reaction to produce DO01 is shown in Figure 1.

### Procedure for treating primed surface

The dried stent was dipped into a solution (Solution A) of "DK01" polymer and Colchicine (a bioactive) and slowly removed to give an even coating of the solution. The sample was initially air dried before being placed in an oven at 75°C for 21 hours.

Solution A: 0.20g of colchicine (as supplied by Sigma-Aldrich Chemical Co) was dissolved in 2-propanol (as supplied by Sigma-Aldrich Chemical Co) to give 10.09g of solution, then 10.11g of DK01a solution (5% in toluene) was added and the resulting solution mixed.

The sample was then immersed in deionised water for 30 seconds, the excess water drained off on a tissue and the sample dried at 50°C for 30 minutes.

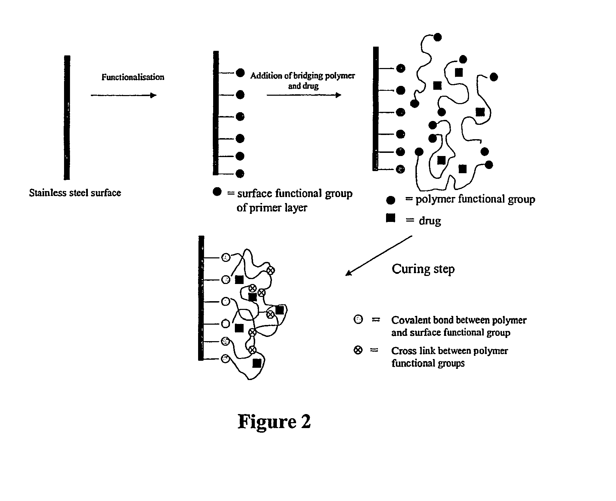

Figure 2 gives a schematic representation of what is thought to happen at the substrate surface. As a result of curing the reactive functional groups of the polymer react with the functionalised surface and also with other functional groups on the molecule.

Without wishing to be constrained by theory, it is thought that unreacted trimethoxysilyl groups on the primed surface hydrolyse to give hydroxyl groups. These then provide a site for the trimethoxysilyl end groups of polymer DK01 to react with. As a less preferred alternative, polymer DK01 could react with pendent hydroxyl or oxide groups on an unprimed surface.

### Procedure for testing drug release properties

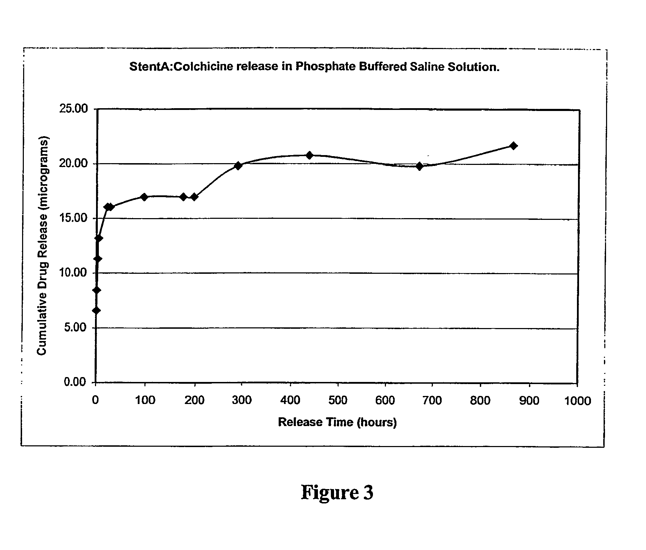

The stent was placed in a tube containing 4mls of Phosphate Buffered Saline solution (prepared from tablets supplied by Sigma-Aldrich Chemical Co by dissolving 1 tablet in200mls of deionised water) and agitated by rolling. The saline solution was sampled at intervals and its colchicine content determined by monitoring its absorbance at 350nm using UV/Vis Spectrometry. A calibration plot for various concentrations of Colchicine (4 to 99 micrograms) in solution against the solution's absorbance at 350nm was constructed to convert sample's release absorbance into drug release values in micrograms per stent. The graph of Colchicine released against release time is plotted in Figure 3. This demonstrates that the DK01 polymer is a suitable material for loading and slow release of Colchicine.

### Example 11

A commercially available stainless steel stent was cleaned and functionalised following the method in sections 1 and 2 of Example 1.

A polymer (DK05) was then prepared as set out below.

### Procedure for synthesis of DK05

| Chemical | Supplier | Catalogue number | Quantity |
|---|---|---|---|
| anhydrous toluene | Aldrich | 24,451 | 119 g (138 ml) |
| poly(propylene glycol) tolylene diisocyanate terminated MW = 2,300 | Aldrich | 43,349-7 | 3.5 g |
| poly(dimethylsiloxane) bis (3-amino propyl) terminated MW = 27,000 | Aldrich | 48,169 | 17.5 g |
| nitrogen | Air Products | | |

| | | | |
|---|---|---|---|
| 1. All glassware was thoroughly dried prior to use. 2. A solution of poly(dimethylsiloxane) bis (3-amino propyl) terminated (17.5 g) in toluene (69 ml) was made up in a flat bottomed flask and purged with nitrogen. The solution was mixed until the polymer was completely dissolved. 3. A solution of poly(propylene glycol) tolylene diisocyanate terminated (3.5 g) in toluene (69 ml) was also made up in a flat bottomed flask and purged with nitrogen. The solution was mixed until the polymer was completely dissolved. 4. The three necked flask was equipped with a dropping funnel, magnetic stirrer bar, nitrogen supply and Dreschel bottle filled with glycerol at the nitrogen outlet. 5. The solution of poly(dimethylsiloxane) bis (3-amino propyl) terminated was added to the flask and the solution of poly(propylene glycol) tolylene diisocyanate terminated was added to the dropping funnel. 6. The solution of poly(propylene glycol) tolylene diisocyanate terminated was added slowly to the solution of poly(propylene glycol) tolylene diisocyanate terminated and the mixing was continued for a further 90 min. 7. The resultant polymer solution was then stored in a flat bottomed flask equipped with a Subaseal under a nitrogen atmosphere. | | | |

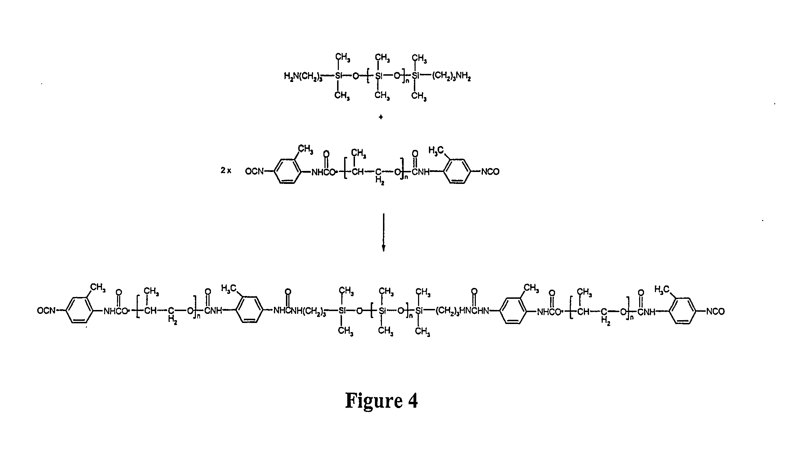

The reaction to produce DK05 is shown in Figure 4.

### Procedure for treating primed surface

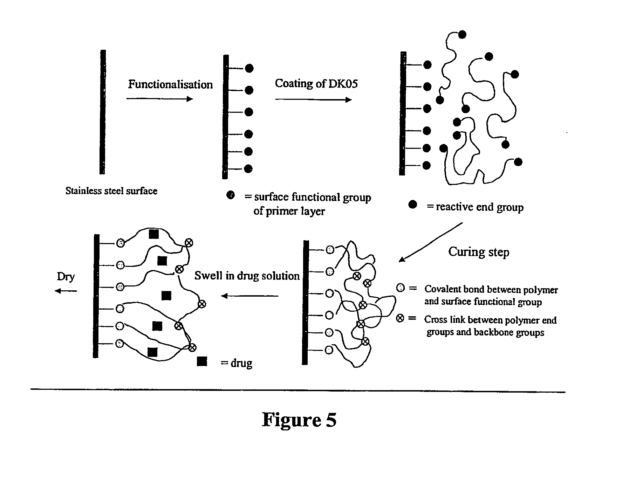

DK05 is coated onto the surface and cured so that the reactive end groups react with the functionalised surface and also with groups in the polymer backbone. The drug is loaded by swelling the polymer with the drug solution and then removing the solvent to leave the drug in the coating. The process is shown schematically in Figure 5, and full details of the process are as follows:

The dried, functionalised stent was dipped into a 15 % w/w solution of DK05 in toluene and slowly removed to give an even coating. The sample was initially air dried before being placed in an oven at 75 °C at reduced pressure (-0.8 mBar) for 24 hours.

The stent was then rinsed by immersing in 3 aliquots of 2-propanol for 3 x 10 min followed by immersing in 3 aliquots of 2-propanol:deionised water (1:1 v/v) for 3 x 10 min. The stent was then dried at 75 °C at reduced pressure (-0.8 mBar) for 24 hours.

The polymer coated stent was placed in a 1% solution of colchicine in toluene : 2-propanol (1:1 v/v) for -2 hr, followed by air drying before being placed in an oven at 75 °C at reduced pressure (-0.8 mBar) for 24 hours. The stent was then rinsed in deionised water for 1 min., followed by drying at 75 °C at reduced pressure (-0.8 mBar) for at least 2 hours.

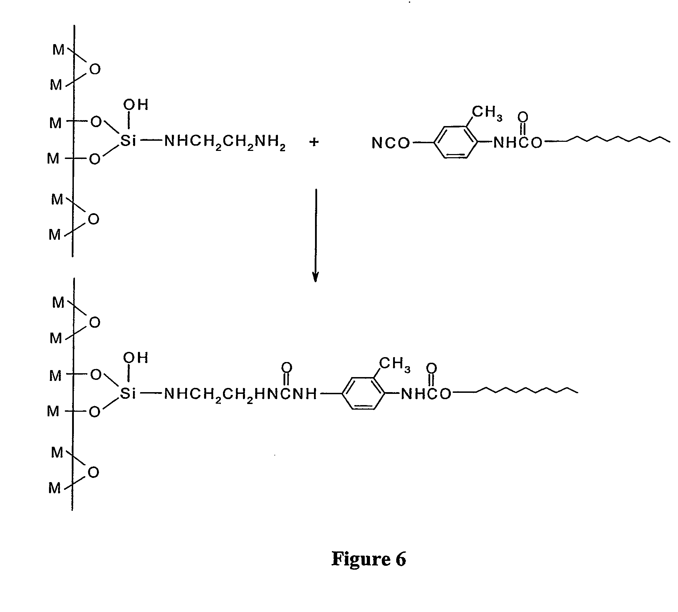

Without wishing to be constrained by theory, it is thought that isocyanate end groups of the polymer react with the amine groups on the primer layer, to bond the polymer covalently to the surface. This is shown in Figure 6, in which the end group of the polymer is shown and not the whole polymer structure..

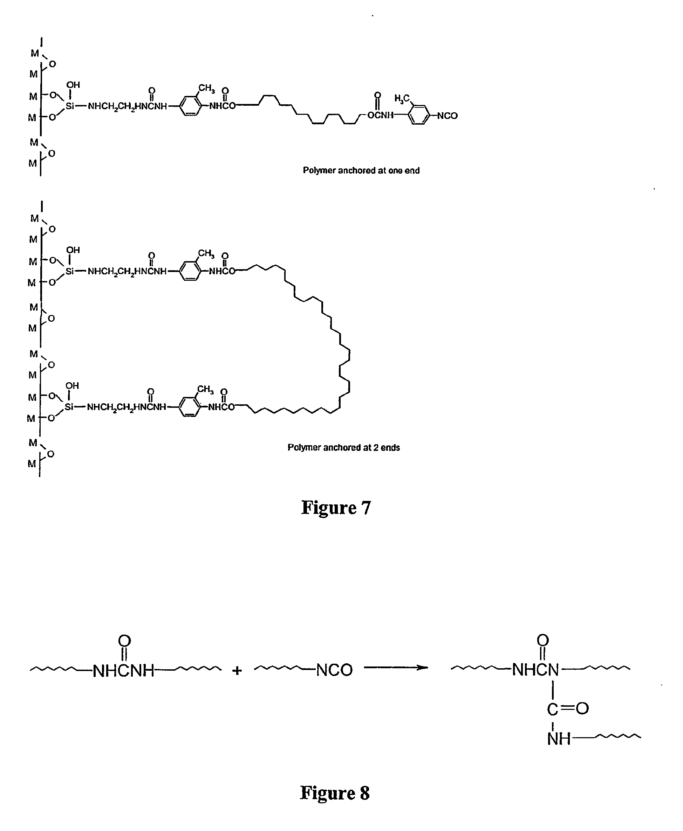

The anchoring of the polymer to the primer layer could be occurring through one end group of the polymer or both end groups could react with the surface as shown in Figure 7.

Once the stent has been coated, the coating is cured at 75 °C for -24 hr. During this curing step, the isocyanate end groups react with urea groups in the polymer chain and this leads to cross-linking *via* biuret groups. This is shown in Figure 8.

### Procedure for testing drug release

### (a) Effect of identity of solvent

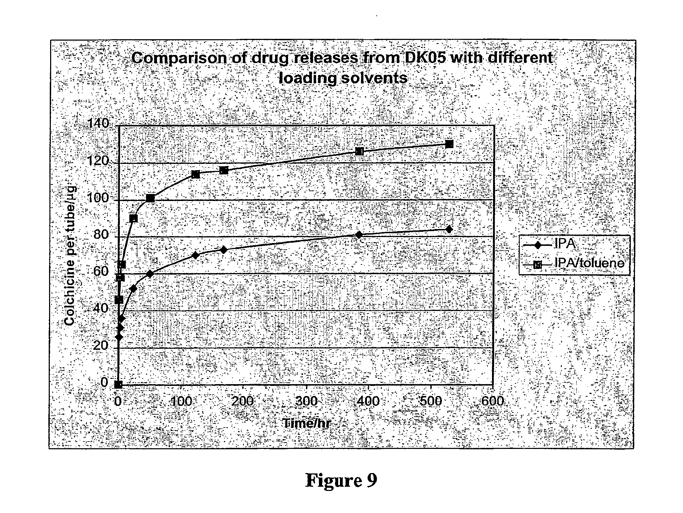

1. 8 Stainless steel heat exchanger tubes were functionalised as described previously.
2. The tubes were dipped in a 5% solution of DK05 in THF and then dried overnight at 75 °C under vacuum.
3. The tubes were rinsed the following day with toluene (15 min), 2-propanol (15 min), deionised water (15 min) and then 2-propanol (5 min). The tubes were air dried over night at room temperature.
4. 4 of the coated tubes were immersed in a 1% solution of colchicine in 2-propanol and and 4 were immersed in a 1% solution of colchicine in 2-propanol:toluene (1:1) for 2 hr.
5. The tubes were then dried overnight at 50 °C and then immersed in deionised water for 30 sec and then dried again at 50 °C for 2-3 hr.
6. Each tube was then placed in 4 ml of phosphate buffered saline (PBS) solution and agitated.
7. The PBS solution was analysed at intervals using UV/VIS spectroscopy. The absorbance of the solution was taken at 354 nm and this absorbance converted to a drug per tube released using a calibration curve. The drug per tube released was plotted against time and this is shown in the graph of Figure 9.

### (b) Effect of concentration of bioactive

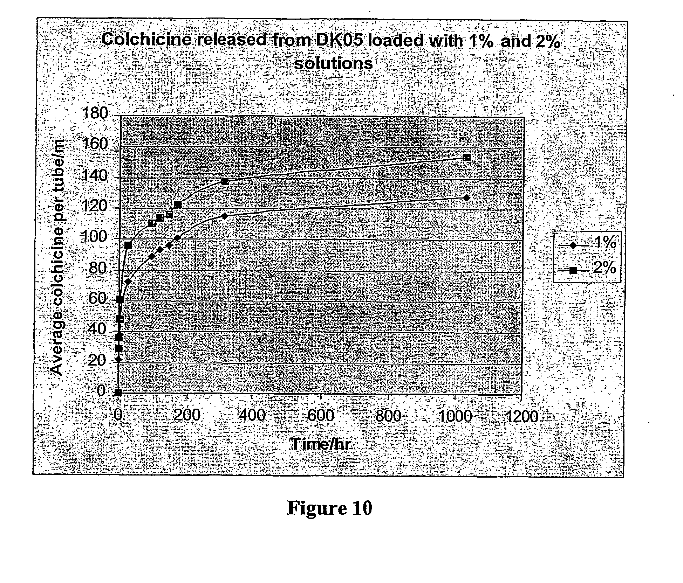

1. 8 Stainless steel heat exchanger tubes were functionalised as described previously.
2. The tubes were dipped in a 5% solution of DK05 in THF and then dried overnight at 75 °C under vacuum.
3. The tubes were rinsed the following day with toluene (15 min), 2-propanol (15 min), deionised water (15 min) and then 2-propanol (5 min). The tubes were dried at 50°C for 2 hr.
4. 4 of the coated tubes were immersed in a 1% solution of colchicine in 2-propanol:toluene (1:1) and 4 of the coated tubes were immersed in a 2% solution of colchicine in 2-propanol:toluene (1:1). The tubes were left in the solutions for 2 hr and then dried overnight at 75 °C under vacuum.
5. The tubes were immersed in deionised water for 1 min and then dried at 75 °C under vacuum for 2.5 hr.
6. Each tube was then placed in 4 ml of phosphate buffered saline (PBS) solution and agitated.
7. The PBS solution was analysed at intervals using UV/VIS spectroscopy. The absorbance of the solution was taken at 354 nm and this absorbance converted to a drug per tube released using a calibration curve. The drug per tube released was plotted against time and this is shown in the graph of Figure 10.

### (c) Effect of number of layers of coating

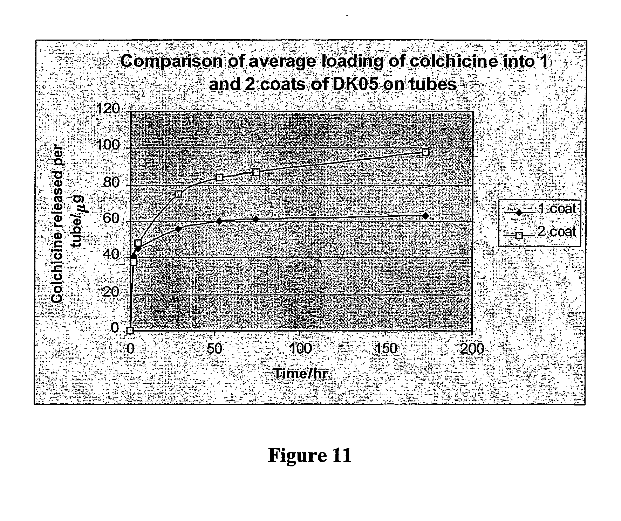

1. 8 Stainless steel heat exchanger tubes were functionalised as described previously.
2. The tubes were dipped in a 5% solution of DK05 in THF and then dried for 2 hr at 75 °C under vacuum.
3. Four of the tubes were given an extra coat at this stage and then all the tubes were dried at 75 °C under vacuum over night.
3. The tubes were rinsed the following day with toluene (15 min), 2-propanol (15 min), deionised water (15 min) and then 2-propanol (5 min). The tubes were dried at 75 °C under vacuum for 2 hr.
4. The tubes were then immersed in a 1% colchicine solution in 2-propanol:toluene (1:1) for 90 min, followed by drying at 75 °C under vacuum over night.
5. The tubes were immersed in deionised water for 1 min and then dried at 75 °C under vacuum for 2.5 hr.
6. Each tube was then placed in 4 ml of phosphate buffered saline (PBS) solution and agitated.
7. The PBS solution was analysed at intervals using UV/VIS spectroscopy. The absorbance of the solution was taken at 354 nm and this absorbance converted to a drug per tube released using a calibration curve. The drug per tube released was plotted against time and this is shown in the graph of Figure 11.

### Example 12

A polymer (DK08) was prepared as set out below.

### Procedure for synthesis of DK08

| Chemical | Supplier | Catalogue number | Quantity |
|---|---|---|---|
| Anhydrous THF | Aldrich | 16,656-2 | 172 ml |
| 3-(triethoxysilyl)propyl isocyanate | Aldrich | 41,336-4 | 7.2 g |
| poly(vinyl butyral-co-vinyl alcohol-co-vinyl acetate) MW = 50,000-80,000 | Aldrich | 18,256-7 | 20.0g |
| nitrogen | Air Products | | |

| | | | |
|---|---|---|---|
| 1. Poly(vinyl butyral-co-vinyl alcohol-co-vinyl acetate) (20 g) was dried over night at 50 °C in a three necked round bottomed flask. 2. THF (172 ml) was added to the polymer and allowed to dissolve over a few hours. 3. The three necked flask was equipped with thermometer, overhead stirrer rod, nitrogen supply and Dreschel bottle filled with glycerol at the nitrogen outlet. The flask was placed in a heating mantle. 4. The solution was stirred with a nitrogen purge whilst 3-(triethoxysilyl)propyl isocyanate (7.2 g) was added. 5. The solution was heated to 30-40 °C for 1.5 hr followed by no heating for 16 hr followed by heating at 30-40 °C for 6 hr. 6. The solution was then stored under nitrogen. | | | |

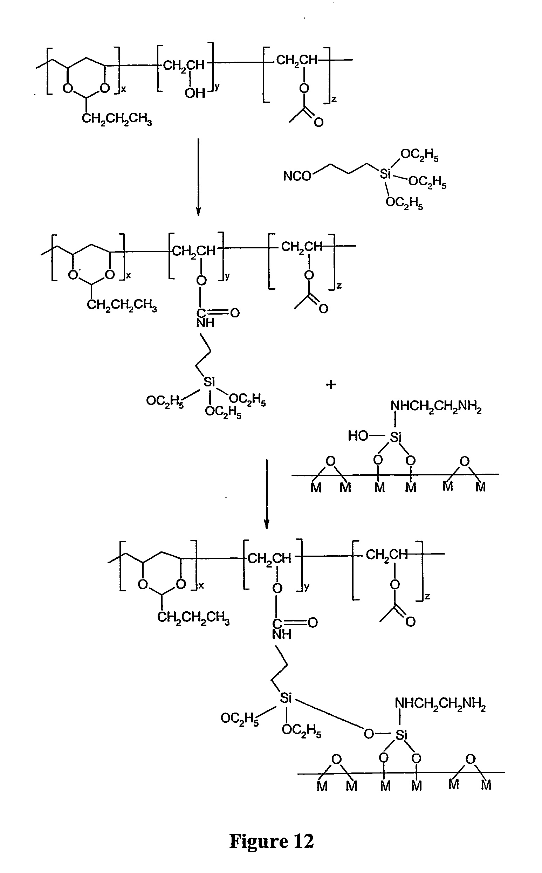

Poly(vinyl butyral-co-vinyl alcohol-co-vinyl acetate) was modified by reacting the hydroxyl group of the vinyl alcohol unit with 3-(triethoxysilyl)propyl isocyanate. This produced a pendant triethoxysilane group to the polymer, which can react with any hydroxyl groups on the surface or cross link with other triethoxysilane groups on other polymer chains. What is thought to be the reaction scheme is shown in Figure 12.

A bioactive can be mixed with the polymer prior to coating. This results in a dried coating on the surface of polymer and bioactive mixed together. When the polymer/ bioactive coating is immersed in an aqueous media, the bioactive leaches out by the aqueous media diffusing into the coating, dissolving the bioactive and then diffusing out.

### Example 13

This system differs from the others described so far as the reactive groups are present on the surface and not on the polymer. The drug is loaded with the polymer and the coating is anchored to the metal by covalent bonding through the triethoxysilyl group on the surface reacting with the hydroxyl group of the polymer. As the polymer is inert, there is no risk of the polymer reacting with the drug during coating.

### Procedure for synthesis of DK09

1. A stainless steel plate was sonicated in 2-propanol for 15 mins and then in deionised water for 15 mins, followed by drying over night at 130 °C.
2. The plate was functionalised as in Example 2.
3. The amino group on the functionalised steel was then reacted with the isocyano group of 3-(triethoxysilyl) isocyanate to form a urea linkage, yielding triethoxysilyl groups on the surface. This was performed by adding the stainless steel plate to a solution of 3-(triethoxysilyl)isocyanate (9 ml) in anhydrous toluene (219 ml). The plate was immersed in the solution for 15 mins under a nitrogen blanket.
4. The plate was then rinsed in anhydrous toluene for 15 mins before being stored in a dessicator under vacuum overnight.
5. The plate was then dip coated in 10 g of a 15 % w/w solution of poly(vinyl butyral-co-vinyl alcohol-co-vinyl acetate) in 2-butanone containing 1 mg of rapamycin.
6. The plate was dried at 75 °C at reduced pressure over night.
7. 20 mg of coating was added to the stainless steel sheet, indicating that 13 µg of drug was present.

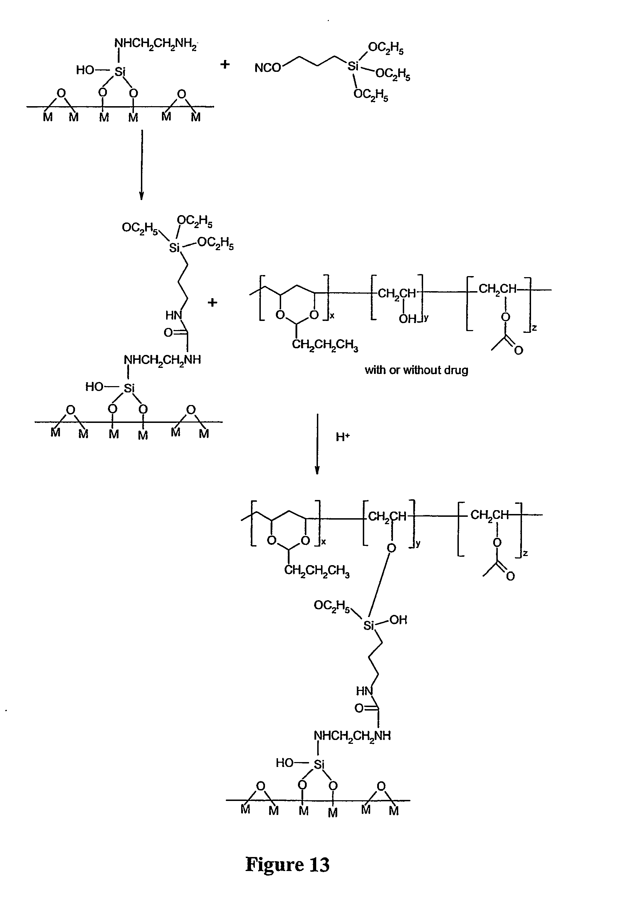

What is thought to be the reaction scheme is shown in Figure 13.

Although drug could shown to be present by stripping the coating from the stainless steel sheet in 2-propanol, no drug was released from the coating into phosphate buffered saline solution.

### Example 14

A metal surface was treated as in Example 13 but with the addition to the coating of a hydrophilic polymer (poly(ethylene glycol)):
1. Stainless steel strips approx 6-8mm in width were cleaned in IPA with ultrasound for 15 mins, followed by drying at 130 °C for 30mins
2. The plate was functionalised as in Example 2.
3. The amino group on the functionalised steel was then reacted with the isocyano group of 3-(triethoxysilyl) isocyanate to form a urea linkage, yielding triethoxysilyl groups on the surface. This was performed by adding the stainless steel plate to a solution of 3-(triethoxysilyl)isocyanate (9 ml) in anhydrous toluene (219 ml). The plate was immersed in the solution for 15 mins under a nitrogen blanket.
4. The plate was then rinsed in anhydrous toluene for 15 mins before being stored in a dessicator under vacuum overnight.
5. A 20 % w/w solution of poly(vinyl butyral-co-vinyl alcohol-co-vinyl acetate) in 2-butanone (solution A) and a 10 % w/w solution of poly(ethylene glycol) in 2-butanone (solution B) were prepared.
6. A formulation (solution C) made of solution A and solution B (4:1 w/w) was prepared and mixed for 30 mins. The final solution had a concentration 15% w/w.
7. Colchicine (30mg) was added to the solution C (2 g) and ultrasonified for 5 mins, to give solution D.
8. The functionalised strips were dipped into solution D and remove at constant rate to give an even coating.
9. The coated strips were held over a hot plate for approx 15-30 secs to prevent evaporative cooling.
10. The strips were left to dry in air for 30 mins
11. The strips were placed in a 50 °C oven for 1 hour
12. The strips were placed in a vacuum oven at 50 °C, -800mbar for 1 hour.
13. Each strip was rinsed in deionised water for 1 minute with 1 change of water.
14. The colchicine was released by placing the strips in 4mls of phosphate buffered saline (PBS) solution, placing on a spiromix and measuring the absorbance at 350nm over a period of 100 hours
15. At the end of this period, the samples were placed in 2-propanol for 10mins with ultrasonification to release the remaining drug

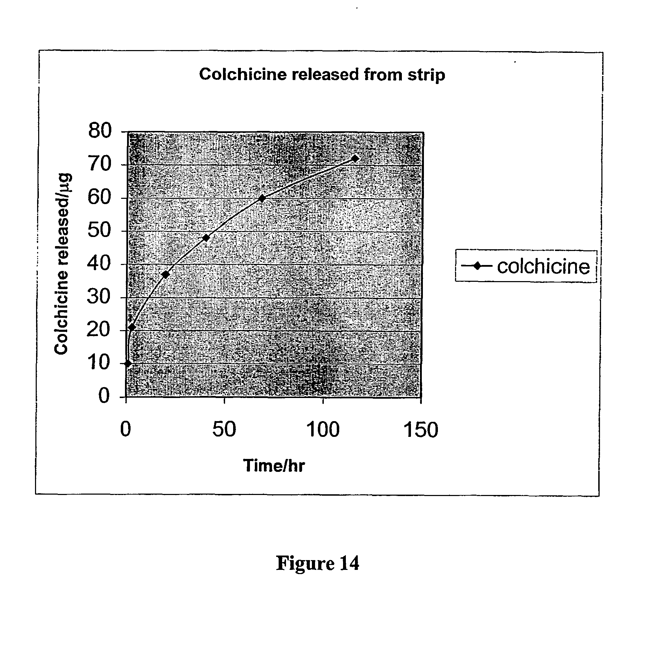

Release profile in PBS solution of a typical strip is shown in Figure 14. The total amount of drug released after sonication in 2-propanol was 320 µg of colchicine

It has been shown that a coating of poly(vinyl butyral-co-vinyl alcohol-co-vinyl acetate) and colchicine but without poly(ethylene glycol) does not release the drug into phosphate buffered saline solution. Stripping the coating from the stainless steel sheet in 2-propanol showed that the drug was present in the coating. The addition of poly(ethylene glycol)increased the hydrophilicity of the coating, which increased the ability of coating to release the drug. This demonstrates how by controlling the hydrophilic/hydrophobic ratio of the coating, the drug release kinetics can be controlled.

### Example 15

This demonstrates the use of THF as an aprotic solvent suitable for functionalisation step 2 in Example 1 by Eosin Y staining of the functionalised layer as in Example 6.

### Cleaning

A stainless steel tube was placed on a suitable holder and placed into a vessel containing 2-propanol. The vessel was placed in an ultrasonic bath (Ultrawave U50 supplied by Ultrawave Limited of Cardiff UK) and sonicated for 15 minutes. The sample was dried for 16 hours at 130°C in an oven.

### Functionalisation

The sample was functionalised as in Section 2 of Example 1, except 190g of Tetrahydrafuran ( HPLC grade, supplied by Sigma Aldrich Chemical Co) was used in place of toluene for the functionalisation solution, and post functionalisation drying was at 50°C for 24hours in an oven.

### Eosin Y Straining

After drying for 2 hours at 50°C the sample was stained with Eosin Y solution, visually examined and then released as detailed in the Eosin Y Coupling section of Example 6.

The absorbance reading for the release solution was 0.19 at 517nm.

This demonstrates the use of Tetrahydrafuran as an aprotic functionalisation solvent.

## Claims

1. A method of treating an article having at its surface oxide or hydroxide, said method comprising the steps of:
priming said surface by contact with an alkoxysilane in an aprotic organic solvent in the presence of an acid catalyst so that the alkoxysilane molecules react with the oxide or hydroxide of said surface to form covalent bonds, the alkoxysilane comprising one or more amino, hydroxyl, carboxylic acid or acid anhydride groups; and
covalently coupling a polymer to said primed surface via said alkoxysilane.

2. A method as claimed in claim 1, wherein the surface is primed by an alkoxysilane of the formula (RO)₃Si(R¹X) wherein R represents methyl, ethyl or propyl and R¹ represents C₂-C₁₀ alkyl in which one or more methylene groups may be replaced by -NH- or -0-, C₂-C₁₀ cycloalkyl or cycloalkylalkyl, C₂-C₁₀ aralkyl or monocyclic or bicyclic aryl and X represents amino, hydroxyl, carboxylic acid or acid anhydride.

3. A method as claimed in claim 2, wherein the alkoxysilane is a compound in which R¹ represents C₂-C₁₀ alkyl in which one or more of the methylene groups is optionally replaced by -NH- and X represents NH₂.

4. A method as claimed in any preceding claim, wherein the alkoxysilane is N-(3-(trimethoxysilyl)propyl)-ethylenediamine or N-(tnethoxysilyl)-ethylenediamine.

5. A method as claimed in any preceding claim, wherein said polymer includes two isocyanate groups.

6. A method as claimed in claim 5, wherein the isocyanate groups are on either end of the polymer.

7. A method as claimed in claim 5 or 6, wherein said polymer is a reaction product of 1 mole of a diamine and two moles of a diisocyanate, with each amine group reacting with an isocyanate group to form a urea linkage.

8. A method as claimed in claim 7, wherein said diamine is a polymer of Formula A:
H₂N-(CH₂)ₘ-Si(R²)₂-O-[Si(R²)₂-O]ₙ-Si(R²)₂-(CH₂)ₘNH₂
wherein:
R² represents an alkyl group having from 1 to 30 carbon atoms, an aryl group, an alkylaryl group, a polyalkylenoxy group, or a halide group,
m is a number from 1 to 12, and
n is a number from 1 to 5,000.

9. A method as claimed in claim 7 or 8, wherein said diisocyanate is a polymer of Formula B:
OCN-R³-NHCO₂-[CHR⁴CH₂-O]ₚ-CONH-R³-NCO
wherein:
R³ represents an alkyl or cycloalkyl group having from 1 to 12 carbon atoms, an aryl group or an alkylaryl group
R⁴ represents hydrogen, methyl, ethyl or propyl, and
p is a number from 1 to 200,000.

10. A method as claimed in claim 9 wherein R³ is alkylphenyl.

11. A method as claimed in any of claims 5 to 8, wherein said diisocyanate is poly[1,4 phenylene diisocyanate-co-poly(1,4-butanediol)] diisocyanate: 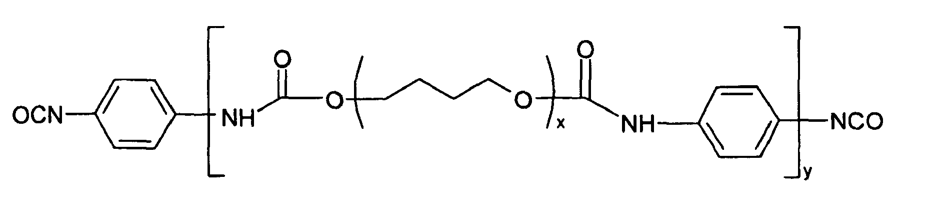
poly(1,4-butanediol),isophorone diisocyanate terminated,
poly(1,4-butanediol), tolylene 2,4-diisocyanate terminated,
poly(ethylene adipate) tolylene 2,4-diisocyanate terminated, or
poly(tetrafluoroethylene oxide-co-difluoromethylene oxide) diisocyanate.

12. A method as claimed in any of claims 1 to 4, wherein said polymer includes at least one pendent alkoxysilane group.

13. A method as claimed in claim 12, wherein said polymer has two alkoxysilane groups, one on each end of the polymer.

14. A method as claimed in claim 13, wherein said polymer is a reaction product of a diisocyanate and a molecule of the formula (RO)₃Si(R¹)NH₂, where R and R¹ are as defined in claim 2.

15. A method as claimed in claim 14, wherein said diisocyanate is a reaction product of 1 mole of a diamine and two moles of a diisocyanate, with each amine group reacting with an isocyanate group to form a urea linkage.

16. A method as claimed in claim 15, wherein said said diamine is a polymer of Formula A and said diisocyanate is a polymer of Formula B as defined in any of claims 8 to 10.

17. A method as claimed in any of claims 14 to 16, wherein R is methyl and R¹ is propyl.

18. A method as claimed in claim 12, wherein said polymer is a reaction product of a molecule of Formula C:
NCO-R⁵-Si(OR⁶)₃
where R⁵ represents an alkyl group having from 1 to 6 carbon atoms and R⁶ represents methyl or ethyl
and a polymer of Formula D:
H₃C-(R⁷)ₓ-(CHOHCH₂)_{y}-(CH₂CHOCOR⁸)_{z}-CH₃
wherein:
R⁷ and R⁸ independently represent alkyl or cycloalkyl of from 1 to 6 carbon atoms or an aryl or alkylaryl, wherein one or more of the carbon atoms of R⁷ or R⁸ may be substituted by O, S or N atoms;
and x, y and z are independently numbers from 1 to 200,000.
the isocyanate group of Formula C reacting with the hydroxyl group of Formula D to form a urethane.

19. A method as claimed in claim 18, wherein R⁵ is propyl and R⁶ is ethyl.

20. A method as claimed in claim 18 or 19, wherein R⁷ represents 2-propyl-4-methyl-1,3-dioxane and R⁸ represents methyl.

21. A method as claimed in claim 18 wherein Formula D is a copolymer of vinyl butyral, vinyl alcohol and vinyl acetate.

22. A method as claimed in any of claims 1 to 4, wherein said polymer is a carbohydrate, polyacrylic acid, polyvinyl alcohol, a hyperbranched polymer, an anti-coagulant, or an antiproliferative agent.

23. A method as claimed in claim 22, wherein said polymer is cellulosic.

24. A method as claimed in claim 23, wherein the alkoxysilane has an amino group and the polymer is carboxymethyl cellulose.

25. A method as claimed in claim 22, wherein said polymer is heparin.

26. A method as claimed in claim 22, wherein the anti-proliferative agent is mitoxantrone, a taxol, a radiolabelled material.

27. A method as claimed in any of claim 1 to 4, wherein
(a) the surface is primed by contact with said alkoxysilane having an amino group,
(b) the primed surface is reacted with a molecule having an isocyanate group and a pendent alkoxysilane group, so that the isocyanate group reacts with said amino group to form a urea linkage, and
(c) a polymer having at least one pendent hydroxyl group is covalently coupled to the surface by reaction between the hydroxyl group and said pendent alkoxysilane group.

28. A method of treating an article having at its surface amino groups, said method comprising the steps of:
(a) reacting the surface with a molecule having an isocyanate group and a pendent alkoxysilane group, so that the isocyanate group reacts with said amino group to form a urea linkage, and
(b) covalently coupling a polymer having at least one pendent hydroxyl group to the surface by reaction between the hydroxyl group and said pendent alkoxysilane group.

29. A method as claimed in claim 27 or 28, wherein the molecule having an isocyanate group and a pendent alkoxysilane group is of Formula C as defined in claim 18 or 19.

30. A method as claimed in any of claims 27 to 29, wherein the polymer having at least one pendent hydroxyl group is of Formula B as defined in claim 18, 20 or 21.

31. A method of treating an article having at its surface amino groups, said method comprising the steps of:
covalently coupling a polymer to said surface wherein the polymer is said polymer as defined in any of claims 5 to 11.

32. A method of treating an article having at its surface oxide or hydroxide, said method comprising the steps of:
either covalently coupling a polymer to said surface,
or priming said surface by contact with an alkoxysilane in an aprotic organic solvent in the presence of an acid catalyst so that the alkoxysilane molecules react with the oxide or hydroxide of said surface to form covalent bonds, and covalently coupling a polymer to said primed surface via said alkoxysilane,
wherein the polymer in either case is said polymer as defined in any of claims 12 to 21.

33. A method as claimed in any preceding claim, wherein a bioactive compound is mixed with said polymer prior to its being coupled to said primed surface.

34. A method as claimed in claim 33 wherein cross-links are formed between functional groups in said polymer after it is coupled to the surface.

35. A method as claimed in any of claims 1 to 32, wherein cross-links are formed between functional groups in said polymer after it is coupled to the surface and then the polymer coating is swollen in a solution of a bioactive compound in order to incorporate the bioactive into the polymer coating.

36. A method as claimed in any of claims 33 to 35 wherein the release characteristics of the bioactive are controlled by incorporating into the surface coating a hydrophilic moiety, a hydrophobic moiety, a copolymer segment, or a combination thereof.

37. A method as claimed in any of claims 33 to 36 wherein said bioactive is an anti-proliferative, an immunosuppresant, an anti-mitotic, an anti-inflammatory, a metalloproteinase inhibitor, an NO donors, an estradiols, an anti-schlerosing agent, a gene, a cell, an anti-sense drug, an anti-neoplastic, an anti-thrombin, or a migration inhibitor.

38. A method as claimed in any of claims 33 to 36 wherein said bioactive is colchicine, rapamycin or mitoxantrone.

39. A method as claimed in any preceding claim, wherein the article is formed of stainless steel or nitanol.

40. A method as claimed in any preceding claim, wherein the article is a coronary stent or a peripheral stent.

## Patentansprüche

1. Verfahren zum Behandeln eines Gegenstands, der an seiner Oberfläche Oxid oder Hydroxid aufweist, wobei das Verfahren die Schritte umfasst:
Grundieren der Oberfläche durch Kontakt mit einem Alkoxysilan in einem aprotischen organischen Lösungsmittel in Gegenwart eines Säurekatalysators, so dass sich die Alkoxysilanmoleküle mit dem Oxid oder Hydroxid der Oberfläche umsetzen, um kovalente Bindungen zu bilden, wobei das Alkoxysilan eine oder mehrere Amino-, Hydroxy-, Carbonsäure- oder Säureanhydridgruppen umfasst; und
kovalentes Kuppeln eines Polymers an die grundierte Oberfläche über das Alkoxysilan.

2. Verfahren gemäß Anspruch 1, wobei die Oberfläche mit einem Alkoxysilan der Formel (RO)₃Si(R¹X) grundiert wird, wobei R Methyl, Ethyl oder Propyl darstellt, R¹ C₂-C₁₀-Alkyl, wobei eine oder mehrere Methylengruppen durch -NH- oder -O- ersetzt sein können, C₂-C₁₀-Cycloalkyl oder -Cycloalkylalkyl, C₂-C₁₀-Aralkyl oder monocyclisches oder bicyclisches Aryl darstellt, und X Amino, Hydroxy, Carbonsäure oder Säureanhydrid darstellt.

3. Verfahren gemäß Anspruch 2, wobei das Alkoxysilan eine Verbindung ist, bei der R¹ C₂-C₁₀-Alkyl darstellt, wobei eine oder mehrere der Methylengruppen gegebenenfalls durch -NH- ersetzt sind, und X NH₂ darstellt.

4. Verfahren gemäß einem der vorstehenden Ansprüche, wobei das Alkoxysilan N-(3-(Trimethoxysilyl)propyl)ethylendiamin oder N-(Triethoxysilyl)ethylendiamin ist.

5. Verfahren gemäß einem der vorstehenden Ansprüche, wobei das Polymer zwei Isocyanatgruppen umfasst.

6. Verfahren gemäß Anspruch 5, wobei die Isocyanatgruppen an beiden Enden des Polymers angeordnet sind.

7. Verfahren gemäß Anspruch 5 oder 6, wobei das Polymer ein Reaktionsprodukt von 1 Mol eines Diamins mit zwei Mol eines Diisocyanats ist, wobei sich jede Amingruppe mit einer Isocyanatgruppe umsetzt, um eine Harnstoffverknüpfung zu bilden.

8. Verfahren gemäß Anspruch 7, wobei das Diamin ein Polymer der Formel A ist:
H₂N-(CH₂)ₘ-Si(R²)₂-O-[Si(R²)₂-O]ₙ-Si(R²)₂-(CH₂)ₘNH₂
wobei:
R² einen Alkylrest mit 1 bis 30 Kohlenstoffatomen, einen Arylrest, einen Alkylarylrest, einen Polyalkylenoxyrest oder eine Halogengruppe darstellt,
m eine Zahl von 1 bis 12 ist, und
n eine Zahl von 1 bis 5.000 ist.

9. Verfahren gemäß Anspruch 7 oder 8, wobei das Diisocyanat ein Polymer der Formel B ist:
OCN-R³-NHCO₂-[CHR⁴CH₂-O]ₚ-CONH-R³-NCO
wobei:
R³ einen Alkyl- oder Cycloalkylrest mit 1 bis 12 Kohlenstoffatomen, einen Arylrest oder einen Alkylarylrest darstellt,
R⁴ Wasserstoff, Methyl, Ethyl oder Propyl darstellt, und
p eine Zahl von 1 bis 200.000 ist.

10. Verfahren gemäß Anspruch 9, wobei R³ Alkylphenyl ist.

11. Verfahren gemäß einem der Ansprüche 5 bis 8, wobei das Diisocyanat Poly[1,4-phenylendiisocyanat-copoly(1,4-butandiol)]diisocyanat: 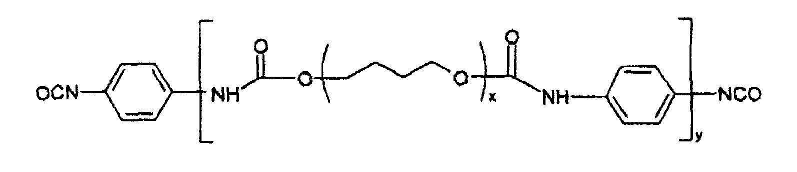 Isophorondiisocyanat-terminiertes Poly(1,4-butandiol), Tolylen-2,4-diisocyanat-terminiertes Poly(1,4-butandiol),
Tolylen-2,4-diisocyanat-terminiertes Poly(ethylenadipat) oder
Poly(tetrafluorethylenoxid-codifluormethylenoxid)diisocyanat ist.

12. Verfahren gemäß einem der Ansprüche 1 bis 4, wobei das Polymer wenigstens einen anhängenden Alkoxysilanrest umfasst.

13. Verfahren gemäß Anspruch 12, wobei das Polymer zwei Alkoxysilanreste aufweist, einen an jedem Ende des Polymers.

14. Verfahren gemäß Anspruch 13, wobei das Polymer ein Reaktionsprodukt eines Diisocyanats und eines Moleküls der Formel (RO)₃Si(R¹)NH₂ ist, wobei R und R¹ wie in Anspruch 2 definiert sind.

15. Verfahren gemäß Anspruch 14, wobei das Diisocyanat ein Reaktionsprodukt von 1 Mol eines Diamins und zwei Mol eines Diisocyanats ist, wobei sich jede Amingruppe mit einer Isocyanatgruppe umsetzt, um eine Harnstoffverknüpfung zu bilden.

16. Verfahren gemäß Anspruch 15, wobei das Diamin ein Polymer der Formel A ist und das Diisocyanat ein Polymer der Formel B ist, wie definiert in einem der Ansprüche 8 bis 10.

17. Verfahren gemäß einem der Ansprüche 14 bis 16, wobei R Methyl ist und R¹ Propyl ist.

18. Verfahren gemäß Anspruch 12, wobei das Polymer ein Reaktionsprodukt eines Moleküls der Formel C:
NCO-R⁵-Si(OR⁶)₃
wobei R⁵ einen Alkylrest mit 1 bis 6 Kohlenstoffatomen darstellt und R⁶ Methyl oder Ethyl darstellt,
und eines Polymers der Formel D:
H₃C-(R⁷)ₓ-(CHOHCH₂)_{y}-(CH₂CHOCOR⁸)_{z}-CH₃
wobei:
R⁷ und R⁸ unabhängig Alkyl oder Cycloalkyl mit 1 bis 6 Kohlenstoffatomen oder ein Aryl oder Alkylaryl darstellen, wobei ein oder mehrere der Kohlenstoffatome von R⁷ und R⁸ durch O-, S- oder N-Atome substituiert sein können;
und x, y und z unabhängig Zahlen von 1 bis 200.000 sind,
ist, wobei sich die Isocyanatgruppe von Formel C mit der Hydroxygruppe von Formel D umsetzt, um ein Urethan zu bilden.

19. Verfahren gemäß Anspruch 18, wobei R⁵ Propyl ist und R⁶ Ethyl ist.

20. Verfahren gemäß Anspruch 18 oder 19, wobei R⁷ 2-Propyl-4-methyl-1,3-dioxan darstellt und R⁸ Methyl darstellt.

21. Verfahren gemäß Anspruch 18, wobei Formel D ein Copolymer von Vinylbutyral, Vinylalkohol und Vinylacetat ist.

22. Verfahren gemäß einem der Ansprüche 1 bis 4, wobei das Polymer ein Kohlenhydrat, eine Polyacrylsäure, Polyvinylalkohol, ein hyperverzweigtes Polymer, ein Antikoagulationsmittel oder ein antiproliferatives Mittel ist.

23. Verfahren gemäß Anspruch 22, wobei das Polymer Cellulose ist.

24. Verfahren gemäß Anspruch 23, wobei das Alkoxysilan eine Aminogruppe aufweist und das Polymer Carboxymethylcellulose ist.

25. Verfahren gemäß Anspruch 22, wobei das Polymer Heparin ist.

26. Verfahren gemäß Anspruch 22, wobei das antiproliferative Mittel Mitoxantron, ein Taxol, ein radioaktiv markiertes Material ist.

27. Verfahren gemäß einem der Ansprüche 1 bis 4, wobei
(a) die Oberfläche durch Kontakt mit dem Alkoxysilan, das eine Aminogruppe aufweist, grundiert wird,
(b) die grundierte Oberfläche mit einem Molekül, das eine Isocyanatgruppe und einen anhängenden Alkoxysilanrest aufweist, umgesetzt wird, so dass sich die Isocyanatgruppe mit der Aminogruppe umsetzt, um eine Harnstoffverknüpfung zu bilden, und
(c) ein Polymer, das wenigstens eine anhängende Hydroxygruppe aufweist, durch Umsetzung zwischen der Hydroxygruppe und dem anhängenden Alkoxysilanrest kovalent an die Oberfläche gekuppelt wird.

28. Verfahren zum Behandeln eines Gegenstands, der an seiner Oberfläche Aminogruppen aufweist, wobei das Verfahren die Schritte umfasst:
(a) Umsetzen der Oberfläche mit einem Molekül, das eine Isocyanatgruppe und einen anhängenden Alkoxysilanrest aufweist, so dass sich die Isocyanatgruppe mit der Aminogruppe umsetzt, um eine Harnstoffverknüpfung zu bilden, und
(b) kovalentes Kuppeln eines Polymers, das wenigstens eine anhängende Hydroxygruppe aufweist, an die Oberfläche durch Umsetzung zwischen der Hydroxygruppe und dem anhängenden Akoxysilanrest.

29. Verfahren gemäß Anspruch 27 oder 28, wobei das Molekül, das eine Isocyanatgruppe und einen anhängenden Alkoxysilanrest aufweist, von der in Anspruch 18 oder 19 definierten Formel C ist.

30. Verfahren gemäß einem der Ansprüche 27 bis 29, wobei das Polymer, das wenigstens eine anhängende Hydroxygruppe aufweist, von der in Anspruch 18, 20 oder 21 definierten Formel B ist.

31. Verfahren zum Behandeln eines Gegenstands, der an seiner Oberfläche Aminogruppen aufweist, wobei das Verfahren die Schritte umfasst:
kovalentes Kuppeln eines Polymers an die Oberfläche, wobei das Polymer das wie in einem der Ansprüche 5 bis 11 definierte Polymer ist.

32. Verfahren zum Behandeln eines Gegenstands, der an seiner Oberfläche Oxid oder Hydroxid aufweist, wobei das Verfahren die Schritte umfasst:
entweder kovalentes Kuppeln eines Polymers an die Oberfläche,
oder Grundieren der Oberfläche durch Kontakt mit einem Alkoxysilan in einem aprotischen organischen Lösungsmittel in Gegenwart eines Säurekatalysators, so dass sich die Alkoxysilanmoleküle mit dem Oxid oder Hydroxid der Oberfläche umsetzen, um kovalente Bindungen zu bilden, und kovalentes Kuppeln eines Polymers an die grundierte Oberfläche über das Alkoxysilan,
wobei das Polymer in beiden Fällen das in einem der Ansprüche 12 bis 21 definierte Polymer ist.

33. Verfahren gemäß einem der vorstehenden Ansprüche, wobei eine bioaktive Verbindung mit dem Polymer gemischt wird, bevor dieses an die grundierte Oberfläche gekuppelt wird.

34. Verfahren gemäß Anspruch 33, wobei Vernetzungen zwischen funktionellen Gruppen des Polymers gebildet werden, nachdem es an die Oberfläche gekuppelt worden ist.

35. Verfahren gemäß einem der Ansprüche 1 bis 32, wobei Vernetzungen zwischen funktionellen Gruppen des Polymers gebildet werden, nachdem es an die Oberfläche gekuppelt worden ist, anschließend wird der Polymerüberzug in einer Lösung einer biologisch aktiven Verbindung gequollen, um den biologisch aktiven Stoff in den Polymerüberzug einzuverleiben.

36. Verfahren gemäß einem der Ansprüche 33 bis 35, wobei die Freisetzungseigenschaften des biologisch aktiven Stoffes durch Einverleiben einer hydrophilen Einheit, einer hydrophoben Einheit, eines Copolymersegments oder einer Kombination davon in den Oberflächenüberzug gesteuert wird.

37. Verfahren gemäß einem der Ansprüche 33 bis 36, wobei der biologisch aktive Stoff ein antiproliferatives Mittel, ein Immunosuppressivum, ein Antimitotikum, ein Entzündungshemmer, ein Metalloproteinasehemmer, ein NO-Donator, ein Estradiol, ein Antisklerosemittel, ein Gen, eine Zelle, ein Antisense-Arzneimittel, ein Antineoplastikum, ein Antithrombin oder ein Migrationshemmer ist.

38. Verfahren gemäß einem der Ansprüche 33 bis 36, wobei der biologisch aktive Stoff Colchicin, Rapamycin oder Mitoxantron ist.

39. Verfahren gemäß einem der vorstehenden Ansprüche, wobei der Gegenstand aus rostfreiem Stahl oder Nitanol gebildet ist.

40. Verfahren gemäß einem der vorstehenden Ansprüche, wobei der Gegenstand ein Koronarstent oder ein peripherer Stent ist.

## Revendications

1. Méthode de traitement d'un article présentant à sa surface un oxyde ou un hydroxyde, ladite méthode se composant des étapes suivantes :
amorçage de ladite surface par contact avec un alkoxysilane dans un solvant organique aprotique en présence d'un catalyseur acide, de sorte que les molécules d'alkoxysilane réagissent avec l'oxyde ou l'hydroxyde de ladite surface pour former des liaisons covalentes, l'alkoxysilane comprenant un ou plusieurs groupements amino, hydroxyle, acide carboxylique ou anhydride d'acide ; et
le couplage covalent d'un polymère à ladite surface amorcée via ledit alkoxysilane.

2. Méthode conforme à la revendication 1, où la surface est amorcée par un alkoxysilane de formule (RO)₃Si(R¹X) où R représente un groupement méthyle, éthyle ou propyle et R¹ représente un groupement alkyle en C₂-C₁₀ où un ou plusieurs groupements méthylène peuvent être remplacés par -NH- ou -O-, cycloalkyle ou cycloalkylalkyle en C₂-C₁₀, arylalkyle ou aryle monocyclique ou bicyclique en C₂-C₁₀ et X représente un groupement amino, hydroxyle, acide carboxylique ou anhydride d'acide.

3. Méthode conforme à la revendication 2, où l'alkoxysilane est un composé où R¹ représente un groupement alkyle en C₂-C₁₀, un ou plusieurs des groupements méthylène étant éventuellement remplacés par -NH-, et X représente NH₂.

4. Méthode conforme à l'une quelconque des revendications précédentes, où l'alkoxysilane est la N-(3-(triméthoxysilyl)propyl,-éthylènediamine ou la N-(triéthoxysilyl)-éthylènediamine.

5. Méthode conforme à l'une quelconque des revendications précédentes, où ledit polymère inclut deux groupements isocyanate.

6. Méthode conforme à la revendication 5, où les groupements isocyanate sont situés de part et d'autre du polymère.

7. Méthode conforme à la revendication 5 ou 6, où ledit polymère est un produit de réaction de 1 mole d'une diamine et de deux moles d'un diisocyanate, chaque groupement amine réagissant avec un groupement isocyanate pour former une liaison urée.

8. Méthode conforme à la revendication 7, où ladite diamine est un polymère de Formule A :
H₂N-(CH₂)ₘ-Si(R²)₂-O-[Si(R²)₂-O]ₙ-Si(R²)₂-(CH₂)ₘNH₂
où :
R² représente un groupement alkyle comportant entre 1 et 30 atomes de carbone, un groupement aryle, un groupement alkylaryle, un groupement polyalkylènoxy ou un groupement halogénure,
m est un nombre compris entre 1 et 12, et
n est un nombre compris entre 1 et 5000.

9. Méthode conforme à la revendication 7 ou 8, où ledit diisocyanate est un polymère de Formule B :
OCN-R³-NHCO₂-[CHR⁴CH₂-O]ₚ-CONH-R³-NCO
où :
R³ représente un groupement alkyle ou cycloalkyle comportant entre 1 et 12 atomes de carbone, un groupement aryle ou un groupement alkylaryle R⁴ représente un atome d'hydrogène ou un groupement méthyle, éthyle ou propyle, et
p est un nombre compris entre 1 et 200 000.

10. Méthode conforme à la revendication 9, où R³ représente un groupement alkylphényle.

11. Méthode conforme à l'une quelconque des revendications 5 à 8, où ledit diisocyanate est le poly[1,4-phénylène diisocyanate-co-poly(1,4-butanediol)]-diisocyanate : 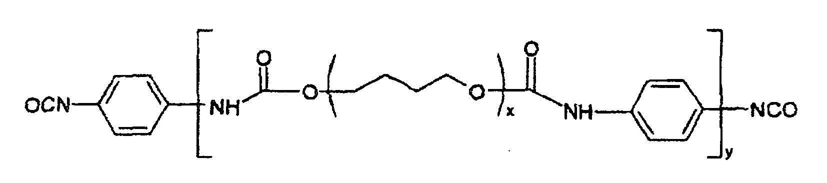 le poly(1,4-butanediol), terminé par isophorone diisocyanate,
le poly(1,4-butanediol), terminé par tolylène 2,4-diisocyanate,
le poly(adipate d'éthylène), terminé par tolylène 2,4-diisocyanate, ou
le poly(tétrafluoroéthylène oxyde-co-difluorométhylène oxyde) diisocyanate.

12. Méthode conforme à l'une quelconque des revendications 1 à 4, où ledit polymère inclut au moins un groupement alkoxysilane pendant.

13. Méthode conforme à la revendication 12, où ledit polymère comporte deux groupements alkoxysilane, de part et d'autre du polymère.

14. Méthode conforme à la revendication 13, où ledit polymère est un produit de réaction d'un diisocyanate et d'une molécule de formule (RO)₃Si(R¹)NH₂, R et R¹ étant tels que définis dans la revendication 2.

15. Méthode conforme à la revendication 14, où ledit diisocyanate est un produit de réaction de 1 mole d'une diamine et de deux moles d'un diisocyanate, chaque groupement amine réagissant avec un groupement isocyanate pour former une liaison urée.

16. Méthode conforme à la revendication 15, où ladite diamine est un polymère de Formule A et ledit diisocyanate est un polymère de Formule B conforme à l'une quelconque des revendications 8 à 10.

17. Méthode conforme à l'une quelconque des revendications 14 à 16, où R représente un groupement méthyle et R¹ représente un groupement propyle.

18. Méthode conforme à la revendication 12, où ledit polymère est un produit de réaction d'une molécule de Formule C :
NCO-R⁵-Si(OR⁶)₃
où R⁵ représente un groupement alkyle comportant entre 1 et 6 atomes de carbone, et R⁶ représente un groupement méthyle ou éthyle
et un polymère de Formule D :
H₃C-(R⁷)ₓ-(CHOHCH₂)_{y}-(CH₂CHOCOR⁸)_{z}-CH)₃
où :
R⁷ et R⁸ représentent indépendamment un groupement alkyle ou cycloalkyle comportant entre 1 et 6 atomes de carbone ou un groupement aryle ou alkylaryle, un ou plusieurs des atomes de carbone de R⁷ ou R⁸ pouvant être substitués par des atomes de O, S ou N ;
et x, y et z représentent indépendamment des nombres compris entre 1 et 200 000.
le groupement isocyanate de Formule C réagissant avec le groupement hydroxyle de Formule D pour former un uréthane.

19. Méthode conforme à la revendication 18, où R⁵ représente un groupement propyle et R⁶ représente un groupement éthyle.

20. Méthode conforme à la revendication 18 ou 19, où R⁷ représente un groupement 2-propyl-4-méthyl-1,3-dioxanne et R⁸ représente un groupement méthyle.

21. Méthode conforme à la revendication 18 où la Formule D représente un copolymère de vinylbutyral, d'alcool vinylique et d'acétate de vinyle.

22. Méthode conforme à l'une quelconque des revendications 1 à 4, où ledit polymère représente un hydrate de carbone, un acide polyacrylique, un alcool polyvinylique, un polymère hyperramifié, un anticoagulant ou un agent antiproliférant.

23. Méthode conforme à la revendication 22, où ledit polymère est cellulosique.

24. Méthode conforme à la revendication 23, où l'alkoxysilane représente un groupement amino et le polymère est la carboxyméthylcellulose.

25. Méthode conforme à la revendication 22, où ledit polymère est l'héparine.

26. Méthode conforme à la revendication 22, où l'agent antiproliférant est la mitoxantrone, un taxol ou une substance radiomarquée.

27. Méthode conforme à l'une quelconque des revendications 1 à 4, où
(a) la surface est amorcée par contact avec ledit alkoxysilane comportant un groupement amino,
(b) la surface amorcée réagit avec une molécule comportant un groupement isocyanate et un groupement alkoxysilane pendant, de sorte que le groupement isocyanate réagisse avec ledit groupement amino pour former une liaison urée, et
(c) un polymère comportant au moins un groupement hydroxy pendant est couplé de façon covalente à la surface par réaction entre le groupement hydroxy et ledit groupement alkoxysilane pendant.

28. Méthode de traitement d'un article présentant à sa surface des groupements amino, ladite méthode se composant des étapes suivantes :
(a) réaction de la surface avec une molécule comportant un groupement isocyanate et un groupement alkoxysilane pendant, de sorte que le groupement isocyanate réagisse avec ledit groupement amino pour former une liaison urée, et
(b) couplage covalent d'un polymère comportant au moins un groupement hydroxy pendant de façon covalente à la surface par réaction entre le groupement hydroxy et ledit groupement alkoxysilane pendant.

29. Méthode conforme à la revendication 27 ou 28, où la molécule comportant un groupement isocyanate et un groupement alkoxysilane pendant est de Formule C comme défini dans la revendication 18 ou 19.

30. Méthode conforme à l'une quelconque des revendications 27 à 29, où le polymère comportant au moins un groupement hydroxyle pendant est de Formule B comme défini dans la revendication 18, 20 ou 21.

31. Méthode de traitement d'un article présentant à sa surface des groupements amino, ladite méthode se composant des étapes suivantes :
couplage covalent d'un polymère à ladite surface, le polymère étant ledit polymère comme défini dans l'une quelconque des revendications 5 à 11.

32. Méthode de traitement d'un article présentant à sa surface un oxyde ou un hydroxyde, ladite méthode se composant des étapes suivantes :
soit le couplage covalent d'un polymère à ladite surface,
soit l'amorçage de ladite surface par contact avec un alkoxysilane dans un solvant organique aprotique en présence d'un catalyseur acide de sorte que les molécules d'alkoxysilane réagissent avec l'oxyde ou
l'hydroxyde de ladite surface pour former des liaisons covalentes, et le couplage covalent d'un polymère à ladite surface amorcée via ledit alkoxysilane,
où le polymère est dans chaque cas ledit polymère comme défini dans l'une quelconque des revendications 12 à 21.

33. Méthode conforme à l'une quelconque des revendications précédentes, où un composé bioactif est mélangé audit polymère avant son couplage à ladite surface amorcée.

34. Méthode conforme à la revendication 33, où des réticulations se forment entre les groupements fonctionnels dans ledit polymère après son couplage à la surface.

35. Méthode conforme à l'une quelconque des revendications 1 à 32, où des réticulations se forment entre les groupements fonctionnels dudit polymère après son couplage à la surface, puis où le revêtement polymère gonfle dans une solution d'un composé bioactif pour incorporer le composé bioactif dans le revêtement polymère.

36. Méthode conforme à l'une quelconque des revendications 33 à 35, où les caractéristiques de libération du composé bioactif sont contrôlées par incorporation dans le revêtement de surface d'un groupement hydrophile, d'un groupement hydrophobe, d'un segment de copolymère ou de l'une de leurs combinaisons.

37. Méthode conforme à l'une quelconque des revendications 33 à 36, où ledit composé bioactif est un agent antiproliférant, immunosuppresseur, antimitotique, anti-inflammatoire, inhibiteur de métalloprotéinase, donneur de NO, de type oestradiol, anti-sclérosant ou un gène, une cellule, un médicament antisens, un agent antinéoplasique, un agent anti-thrombine ou un inhibiteur de migration.

38. Méthode conforme à l'une quelconque des revendications 33 à 36 où ledit composé bioactif est la colchicine, la rapamycine ou la mitoxantrone.

39. Méthode conforme à l'une quelconque des revendications précédentes, où l'article est formé d'acier inoxydable ou de nitanol.

40. Méthode conforme à l'une quelconque des revendications précédentes, où l'article est un stent coronarien ou un stent périphérique.
